# EUROPÄISCHE PATENTANMELDUNG

(11) **EP 0 545 120 A2**
(43) Veröffentlichungstag der Anmeldung: **09.06.1993**
(21) Anmeldenummer: 92119436.1
(22) Anmeldetag: 13.11.1992
(51) Int. Cl.: A61K 31/445, A61K 31/505, A61K 31/54, A61K 31/425

(54) **Kombination von 5-HT2-Rezeptorantagonisten und 5-HT1A-Rezeptoragonisten mit neuroprotektiver Wirkung**

(30) Priorität: 26.11.1991 DE 4138756
(71) Anmelder: Troponwerke GmbH & Co. KG, D-51063 Köln (DE)
(72) Erfinder: Bode-Greuel, Kerstin, Dr., W-5204 Lohmar (DE)
(74) Vertreter: Dänner, Klaus, Dr.

(57) **Zusammenfassung**

Die Wirkung von neuroprotektiven 5-HT₂-rezeptorantagonistischen 4-Fluor-phenyl-Verbindungen wird synergistisch durch 5-HT_{1A}-rezeptoragonistischen Aminomethyl-Chromane verstärkt.

Die Kombination von 5-HT_{1A}-rezeptoragonistischen Aminomethyl-Chromanen und 5-HT₂-rezeptorantagonistischen 4-Fluor-phenyl-Verbindungen eignet sich zur Behandlung von cerebralen Ischämien.

## Beschreibung

Die Erfindung betrifft eine synergistische Kombination von 5-HT_{1A}-rezeptoragonistischen Aminomethyl-Chromanen mit 5-HT₂-rezeptorantagotuslischen 4-Fluor-phenyl-Verbindungen, ihre Herstellung sowie ihre Verwendung in neuroprotektiven Arzneimitteln, insbesondere zur Behandlung von Zuständen der cerebralen Ischämie.

Cerebrale Durchblutungsstörungen haben ein Absterben von Gehirnzellen zur Folge. Diese neuronale Degeneration, die oft erst verzögert eintritt, führt zu Funktionsausfällen im Gehirn mit neurologischen und/oder psychischen Symptomen [Journal of Cerebral Blood Flow and Metabolism 1, 155-185 (1981)].

Die Ursachen für cerebrale Durchblutungsstörungen können durch Arteriosklerose bedingte Gefäßverschlüsse, Hirnblutungen u.a. nach Gefäßriß bei Bluthochdruck, aber auch Ischämien durch Blutdruckabfall oder Embolie sein.

Es ist bekannt, daß 5-HT_{1A}-rezeptoragonistische Wirkstoffe sowohl bei einer prophylaktischen, als auch bei einer nach der cerebralen Ischämie erfolgten Behandlung die neuronale Degeneration und die in Folge auftretenden Funktionsausfälle des Gehirns vermindern [DE-OS 35 43 794].

Es wurde gefunden, daß 5-HT_{1A}-rezeptoragonistische Aminomethyl-Chromane [Komponente A] überraschenderweise die neuroprotektive Wirkung von 5-HT₂-rezeptorantagonistischen 4-Fluor-phenyl-Verbindungen in synergistischer Weise verstärken.

Unter 5-HT_{1A}-rezeptoragonistischen Amminomethyl-Chromanen (Komponente A) im Rahmen der Erfindung werden serotonin-agonistische Wirkstoffe der allgemeinen Formel (I) 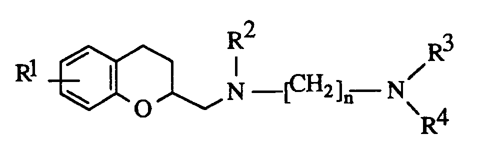
in welcher
- R¹: für Wasserstoff oder Alkoxy mit bis zu 4 Kohlenstoffatomen steht,
- R²: für Wasserstoff oder Alkyl mit bis zu 4 Kohlenstoffatomen steht,
- R³: für Wasserstoff oder Alkyl mit bis zu 4 Kohlenstoffatomen steht,
- R⁴: für eine Gruppe SO₂R⁵ oder 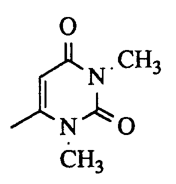 steht, wobei
- R⁵: Alkyl mit bis zu 4 Kohlenstoffatomen bedeutet oder Phenyl oder Naphthyl bedeutet, das durch Halogen oder Alkyl mit bis zu 4 Kohlenstoffatomen substituiert sein kann
oder
- R³ und R⁴: gemeinsam mit dem Stickstoffatom einen Ring der Formel 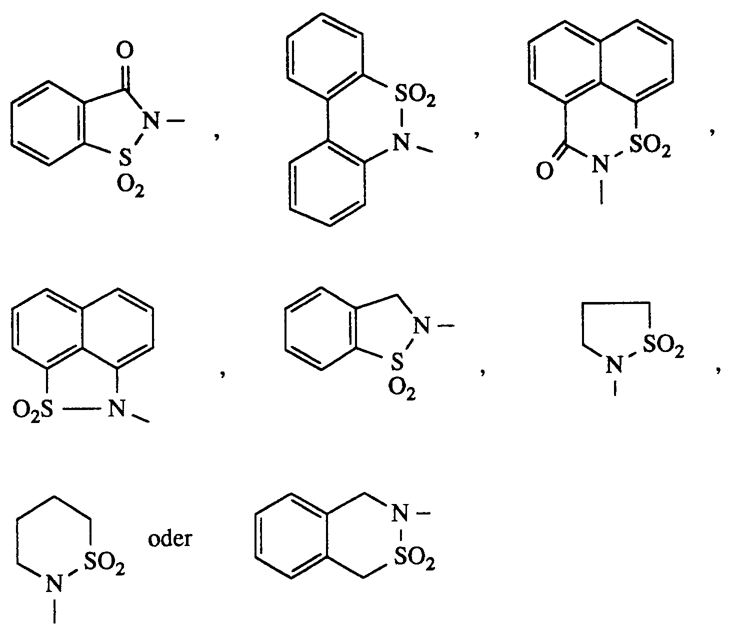 bilden,
- n: für eine Zahl 2, 3, 4 oder 5 steht,
gegebenenfalls in einer isomeren Form, sowie deren Salze verstanden, die bei einer Bindung an 5-HT_{1A}-Rezeptoren eine Bindungsstärke von Kleiner als 1 000 nmol/l aufweisen.

Bevorzugt sind Aminomethyl-Chromane der Formel (I), die bei einer Bindung an 5-HT_{1A}-Rezeptoren eine Bindungsstärke von kleiner als 100 nmol/l aufweisen.

Solche Wirkstoffe können im Adenylat-Cyclasetest (EC₅₀-Wert) identifiziert werden [J. Pharmacol Exp. Ther. 238, 248-253 (1986)]. 5-HT_{1A}-Liganden mit agonistischer bzw. partiell agonistischer Wirkung inhibieren die Forskolin-stimulierte Adenylat-Cyclase. Wirkstoffe, die die Enzymaktivität vermindern, haben eine Serotonin-agonistische bzw. partiell Serotonin-agonistische Wirkung. Der genannte Adenylat-Cyclasetest kann beispielsweise wie folgt durchgeführt werden:
Rattenhippocampus-Membranen werden unter geeigneten Bedingungen mit α-³²P-ATP und Forskolin in Ab- und Anwesenheit von erfindungsgemäßen Verbindungen inkubiert. Nach Abstoppen der Reaktion wird das radioaktiv markierte cycloAMP isoliert und quantitativ bestimmt. Daraus wird die Enzymaktivität berechnet.

Die Bindungsstärke (Inhibitionskonstante bzw. Kᵢ-Wert) ist ein Maß für die Wechselwirkungen zwischen einem Wirkstoff und den 5-HT_{1A}-Rezeptoren [Molecular Pharmacology 16, 687-699 (1979); J. Neurochem. 36, 220-226, (1981)].

Die Bindungsstärke kann beispielsweise wie folgt bestimmt werden:
Kalbshippocampus-Membranen werden mit ³H-Serotonin in An- und Abwesenheit von zu untersuchenden Substanzen inkubiert. Die Reaktion wird durch Filtration gestoppt und die auf den Filtern verbleibende Radioaktivität gemessen. Aus den erhaltenen Verdrängungskurven werden IC₅₀-Werte bzw. Inhibitionskonstanten Kᵢ berechnet.

Bevorzugt sind Aminomethyl-Chromane der allgemeinen Formel (I) in welcher
- R¹: für Wasserstoff oder Methoxy steht,
- R²: für Wasserstoff oder Propyl steht,
- R³: für Wasserstoff oder Methyl steht,
- R⁴: für eine Gruppe -SO₂R⁵ steht,
wobei
- R⁵: Phenyl bedeutet, das gegebenenfalls durch Fluor oder Chlor substituiert ist,
oder
- R³ und R⁴: gemeinsam mit dem Stickstoffatom eine Gruppe der Formel 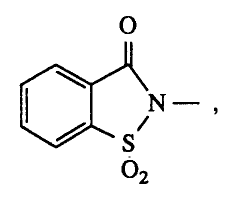 bilden,
und
- n: eine Zahl 4 bedeutet
gegebenenfalls in einer isomeren Form sowie deren Salze.

Besonders bevorzugt sind folgende Aminomethyl-Chroman-Verbindungen:
(+)-, (-)- und (±)-2-[N-[4-(1,1-Dioxido-3-oxo-2,3-dihydro-benzisothiazol-2-yl)butyl]aminomethyl]-8-methoxy-chroman sowie (+)-, (-)- und (±)-2-[N-[4-(1,1-Dioxido-3-oxo-2,3-dihydro-benzisothiazol -2-yl)butyl]aminomethyl]-chroman sowie deren Salze.

Unter 5-HT₂-rezeptorantagonistischen 4-Fluor-phenyl-Verbindungen (Komponente B) im Rahmen der Erfindung werden serotonin-antagonistische Wirkstoffe der allgemeinen Formel (II) 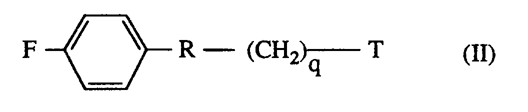
worin
- R: für eine Gruppe der Formel 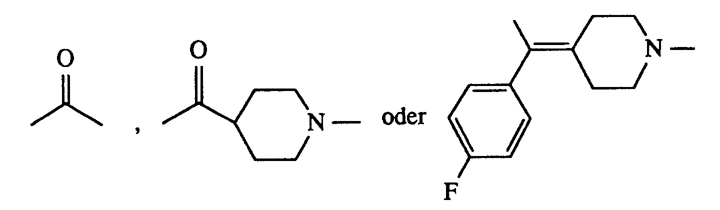 steht,
- T: für eine Gruppe der Formel 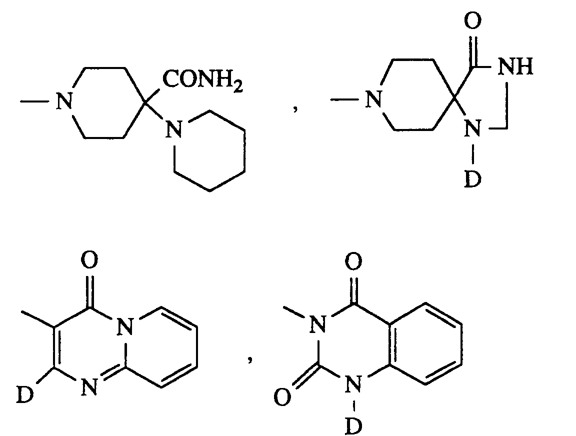 oder 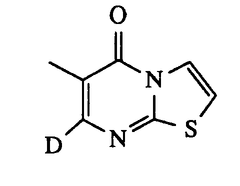 steht,
worin
- D: Wasserstoff, C₁-C₄-Alkyl oder Phenyl bedeutet
und
- q: für eine Zahl 1, 2, 3 oder 4 steht
sowie deren Salze verstanden die bei der Bindung an 5-HT₂-Rezeptoren eine Bindungsstärke von Kleiner als 1000 nmol/l, bevorzugt von 0,1 bis 100 nmol/l, insbesondere Kleiner 10 nmol/l, aufweisen.

Solche Wirkstoffe können im Phosphoinositol-Umsatz-Test untersucht werden [J. Pharmacol. Exp. Ther. 244, 1051-1056 (1988)]. 5-HT₂-Liganden mit antagonistischer Wirkung inhibieren den Serotonin-stimulierten Phosphoinositol-Umsatz. Der gesamte Test kann beispielsweise wie folgt durchgeführt werden:
Cortex-Schnitte 8-Tage-alter Ratten werden unter geeigneten Bedingungen mit ³H-myo-Inositol und Serotonin in An- und Abwesenheit von den zu untersuchenden Verbindungen inkubiert. Nach Abstoppen der Reaktion werden die radioaktiv markierten Inositolphosphate isoliert und quantitativ bestimmt.

Bevorzugt als Komponente B seien folgenden Wirkstoffe genannt:
1-(4-Fluorphenyl)-4-(4-piperidino-4-carbamoylpiperidino)-1-butanon (INN: Pipamperon); 4-Phenyl-8-[3-(4-fluorbenzoyl)propyl]-1-oxo-2,4,8-triazaspiro[4,5]decan (INN: Spiperon), 3-[2-[4-(4-Fluorbenzoyl)-1-piperidinyl]-ethyl]-2,4[1H, 3H]-quinazolinedion (INN: Ketanserin), Pirenperin (INN), Ritanserin (INN) und Pelanserin (INN).

Insbesondere bevorzugt ist Ketanserin.

Die 4-Fluorphenyl-Verbindungen der Formel (II) sind an sich bekannt [EP 13 612; BE 610 830; US 3 155 669; US 3 155 670; US 3 161 644; EP 37 265] und können nach den dort beschriebenen Verfahren hergestellt werden.

Wie bereit beschrieben, verstarken die 5-HT_{1A}-rezeptoragonistischen Aminomethyl-Chromane der Formel (I) in synergistischer Weise die neuroprotektive Wirkung der 5-HT₂-rezeptorantagonistischen 4-Fluor-phenyl-Verbindungen der Formel (II) in der Weise, daß sogar bei Mengen, bei denen der 5-HT-_{1A}-Agonist keine neuroprotektive Wirkung zeigt, durch Kombination mit einem geeigneten 5-HT₂-Antagonisten, der ebenfalls keine oder nur geringe neuroprotektive Wirkung hat, sowohl bei prophylaktischer als auch bei einer nach der cerebralen Ischämie erfolgenden Behandlung die neuronale Degeneration und die in Folge auftretenden Funktionsausfälle des Gehirns deutlich vermindert.

Bezogen auf einen Gewichtsteil des 5-HT_{1A}-agonistischen Wirkstoffes (Komponente A) können 0,01 bis 100 Gewichtsteile, bevorzugt 0,1 bis 10 Gewichtsteile des 5-HT₂-antagonistischen Wirkstoffes (Komponente B) eingesetzt werden.

Besonders gute neuroprotektive Wirkung haben Kombinationen (+); (-)- und (±)-2-[N-[4-(1,1-Dioxido-3-oxo-2,3-dihydro-benzisothiazol-2-yl)butyl]aminomethyl]-8-methoxy-chroman und (+)-, (-)- und (±)-2-[N-[4-(1,1-Dioxido-3-oxo-2,3-dihydro-benzisothiazol -2-yl)butyl]aminomethyl]-chroman als Komponente A mit Ketanserin als Komponente B.

Besonders gute neuroprotektive Wirkung hat die Kombination von (+)-, (-)- und (±)-2-[N-[4-(1,1-Dioxido-3-oxo-2,3-dihydro-benzisothiazol-2-yl)butyl]aminomethyl]-chroman mit Ketanserin, ganz besonders bevorzugt in einem Mengenverhältnis von 1 bis 10 Gewichtsteilen Ketanserin zu 0,02 bis 2 Gewichtsteilen (+)-, (-)- und (±)-2-[N-[4-(1,1-Dioxido-3-oxo-2,3-dihydro-benzisothiazol-2-yl)butyl]aminomethyl]-chroman. Sie ist deshalb ganz besonders bevorzugt.

Die Kombination kann hergestellt werden, indem man die Einzelkompenenten in diese auflösende inerte Lösemittel auflöst und gegebenenfalls nach Abdampfen des Lösemittels die Kombination in üblicher Weise mit Hilfsstoffen vermischt. Als inerte Lösemittel seien beispielhaft Alkohole wie Ethanol oder Polyethylenglykol genannt. Die Komponenten können auch als Feststoff gemischt werden.

Zur vorliegenden Erfindung gehören auch pharmazeutische Zubereitungen, die neben inerten, nichttoxischen, pharmazeutisch geeigneten Hilfs- und Trägerstoffen die erfindungsgemäße Kombination enthalten, die aus der erfindungsgemäßen Kombination bestehen, sowie Verfahren zur Herstellung dieser Zubereitungen.

Die Kombination soll in diesen Zubereitungen in einer Konzentration von 0,1 bis 99,5 Gew.-%, bevorzugt von 0,5 bis 95 Gew.-%, der Gesamtmischung vorhanden sein.

Neben der Kombination können die pharmazeutischen Zubereitungen auch andere pharmazeutische Wirkstoffe enthalten.

Die oben aufgeführten pharmazeutischen Zubereitungen können in üblicher Weise nach bekannten Methoden hergestellt werden, beispielsweise mit dem oder den Hilfs- oder Trägerstoffen.

Im allgemeinen hat es sich als vorteilhaft erwiesen, die Kombination in Gesamtmengen von etwa 0,01 bis etwa 100 mg/kg, bevorzugt in Gesamtmengen von etwa 1 mg/kg bis 50 mg/kg, Körpergewicht je 24 Stunden, gegebenenfalls in Form mehrerer Einzelgaben zur Erzielung des gewünschten Ergebnisses zu verabreichen.

Es kann aber gegebenenfalls vorteilhaft sein, von den genannten Mengen abzuweichen, und zwar in Abhängigkeit von der Art und vom Körpergewicht des behandelten Objekts, vom individuellen Verhalten gegenüber dem Medikament der Art und Schwere der Erkrankung, der Art der Zubereitung und Applikation, sowie dem Zeitpunkt bzw. Intervall, zu welchem die Verabreichung erfolgt.

Die Wirkungsweise der erfindungsgemäßen Kombination zur präventiven und nachfolgenden Behandlung von cerebralen Ischämien lassen sich anhand des Gerbil-Modells (Wüstenrennmäuse-Modell) der transienten globalen Vorderhirnischämie nachweisen.

### Prüfmethoden

Für die Auslösung einer transienten Vorderhirnischämie wurden bei mongolischen Wüstenrennmäusen (Gerbil) beide Halsschlagadern 5 Minuten lang unter Halothannarkose (1 %ig in Raumluft) abgeklemmt. Sieben Tage nach der Ischämie wurden die Tiere transkardial zwecks Paraffineinbettung der Gehirne perfundiert. Es wurden 7 µm dicke Frontalschnitte der Gehirne hergestellt. Aus 4 verschiedenen Ebenen wurde jeweils ein Schnitt mikroskopisch mittels einer Camera-lucida-Projektion ausgewertet. Die im CA1-Bereich überlebenden Pyramidenzellen wurden innerhalb standardisierter Rahmen gezählt. Für jedes Tier wurden sämtliche Zahlen addiert. Die Signifikanz der unterschiedlichen Durchschnittswerte wurde durch eine Varianzanalyse nach Scheffe ermittelt. P<0,05 wurde als Signifikanzniveau festgelegt.

15 Minuten vor Eintritt der Ischämie und zweimal täglich an den 3 folgenden Tagen wurden Medikamente intraperitoneal in 300 µl 0,9 %igern NaCl injiziert.

### Test-Daten

Ketanserin wurde in einer Dosis von 5 mg/kg verabreicht, die eine submaximale Wirkung mit einem 52 %igen Schutz der Pyramidenzellen (s. Zeichnung) hervorrief. Durch Zusatz zunehmender Dosen (-)-2-[N-[4-(1,1-Dioxido-3-oxo-2,3-dihydro-benzisothiazol-2-yl)-butyl]aminomethyl ]chroman wurde die neuroprotektive Wirkung von Ketanserin, selbst bei einer Dosis (0,1 mg/kg), bei der (-)-2-[N-[4-(1,1-Dioxido-3-oxo-2,3-dihydro-benzisothiazol-2-yl)-butyl]aminomethyl]chroman unwirksam war, weiter verbessert.
(*P <0,5; **P <0,01: *** P <0,001)

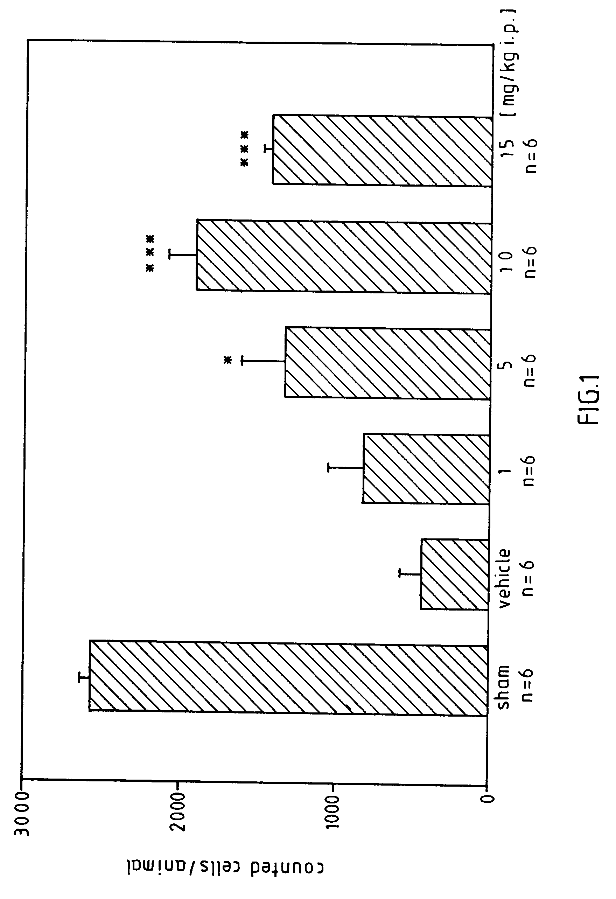

- Fig. 1:: Ketanserin (5-HT₂-Antagonist)

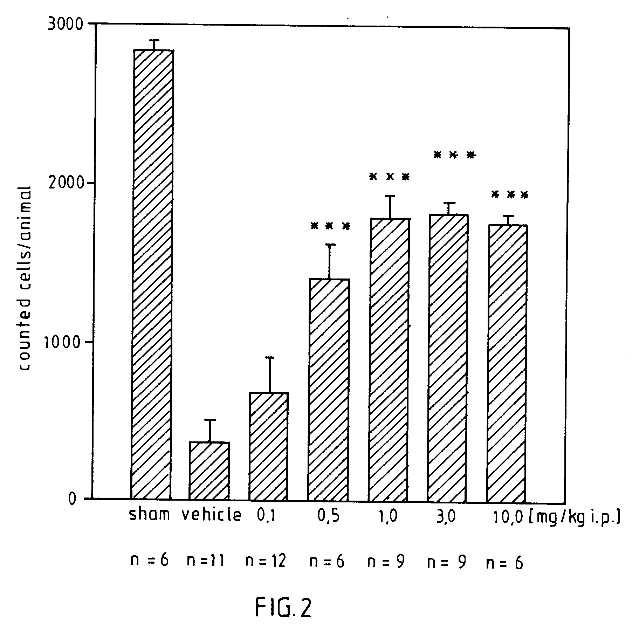

- Fig. 2:: (-)-2-[N-[4-(1,1-Dioxido-3-oxo-2,3-dihydro-benzisothiazol-2-yl)-butyl]-aminomethyl]chroman (5-HT_{1A}-Antagonist)

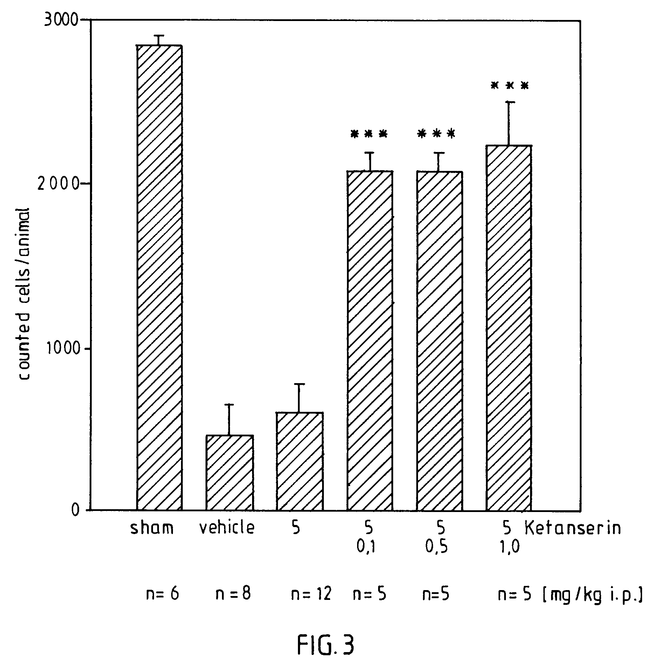

- Fig. 3:: Kombination von (-)-2-[N-[4-(1,1-Dioxido-3-oxo-2,3-dihydro-benzisothiazol-2-yl)-butyl]-aminomethyl]chroman mit Ketanserin

## Patentansprüche

1. Kombinationspräparat enthaltend mindestens ein Aminomethyl-Chroman der allgemeinen Formel 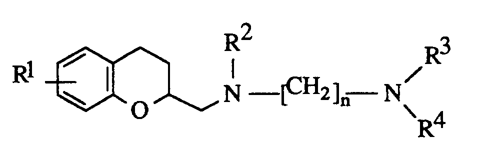 in welcher
R¹ für Wasserstoff oder Alkoxy mit bis zu 4 Koblenstoffatomen steht,
R² für Wasserstoff oder Alkyl mit bis zu 4 Kohlenstoffatomen steht,
R³ für Wasserstoff oder Alkyl mit bis zu 4 Kohlenstoffatomen steht,
R⁴ für eine Gruppe SO₂R⁵ oder 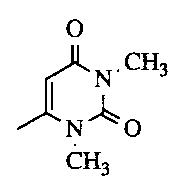 steht,
wobei
R⁵ Alkyl mit bis zu 4 Kohlennstoffatomen bedeutet oder Phenyl oder Naphthyl bedeutet, das durch Halogen oder Alkyl mit bis zu 4 Kohlenstoffatomen substituiert sein kann
oder
R³ und R⁴ gemeinsam mit dem Stickstoffatom einen Ring der Formel 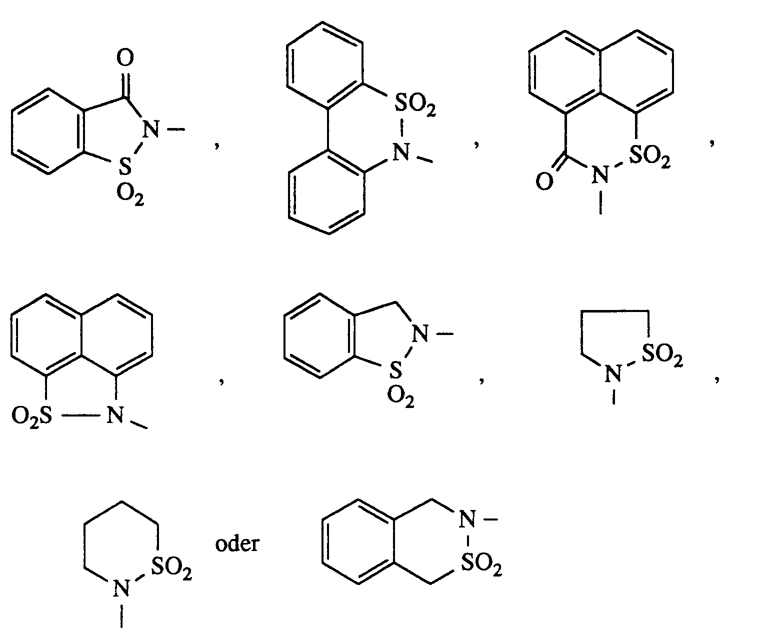 bilden,
n für eine Zahl 2, 3, 4 oder 5 steht,
gegebenenfalls in einer isomeren Form oder deren Salze als Komponente A und mindestens eine 4-Fluor-phenyl-Verbindung der allgemeinen Formel 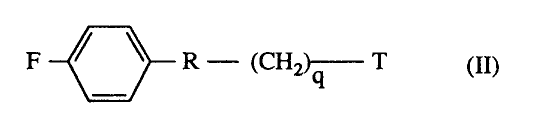 worin
R für eine Gruppe der Formel 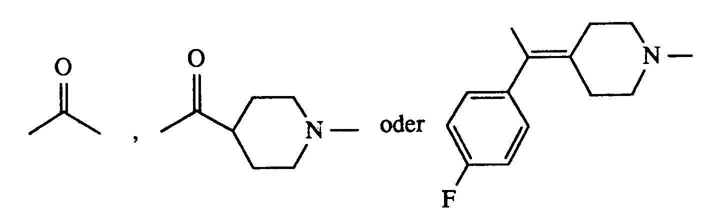 steht,
T für eine Gruppe der Formel 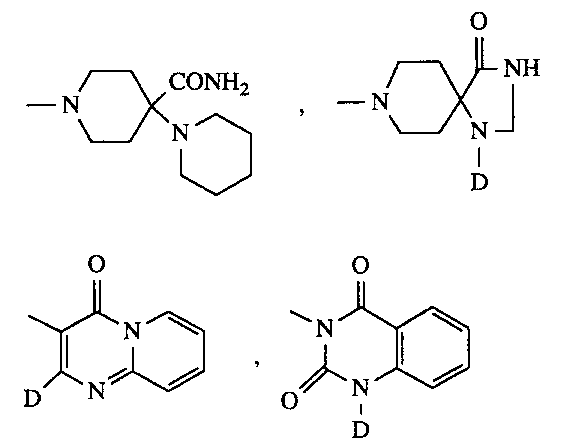 oder 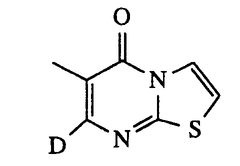 steht,
worin
D Wasserstoff, C₁-C₄-Alkyl oder Phenyl bedeutet
und
q für eine Zahl 1, 2, 3 oder 4 steht
oder deren Salze als Komponente B.

2. Kombinationspräparat nach Anspruch 1, dadurch gekennzeichnet, daß als Komponente A Aminomethyl-Chromane der allgemeinen Formel (I),
worin
R¹ für Wasserstoff oder Methoxy steht,
R² für Wasserstoff oder Propyl steht,
R³ für Wasserstoff oder Methyl steht,
R⁴ für eine Gruppe -SO₂R⁵ steht,
wobei
R⁵ Phenyl bedeutet, das gegebenenfalls durch Fluor oder Chlor substituiert ist,
oder
R³ und R⁴ gemeinsam mit dem Stickstoffatom eine Gruppe der Formel 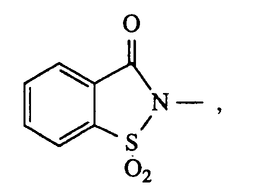 bilden,
und
n eine Zahl 4 bedeutet
gegebenenfalls in einer isomeren Form oder deren Salze, eingesetzt wurden.

3. Kombinationspräparat nach Anspruch 1 dadurch gekennzeichnet, daß als Komponente A (+)-, (-)- oder (±)-2-[N-[4-(1,1-Dioxido-3-oxo-2,3-dihydro-benzisothiazol-2-yl)butyl]aminomethyl]-8-methoxy-chroman oder (+)-, (-)-oder (±)-2-[N-[4-(1,1-Dioxido-3-oxo-2,3-dihydro-benzisothiazol-2-yl)butyl]-aminomethyl]-chroman oder ein Salz davon eingesetzt wird.

4. Kombinationspräparat nach Anspruch 1, wobei als Komponente B 1-(4-Fluorphenyl)-4-(4-piperidino-4-carbamoylpiperidino)-1-butanon (INN: Pipamperon), 4-Phenyl-8-[3-(4-fluorbenzoyl)propyl]-1-oxo-2,4,8-triazaspiro-[4,5]decan (INN: Spiperon),3-[2-[4-(4-Fluorbenzoyl)-1-piperidinyl]-ethyl]-2,4[1H, 3H]-quinazolinedion (INN: Ketanserin), Pirenperin (INN), Ritanserin (INN) oder Pelanserin (INN) eingesetzt wird.

5. Kombinationspräparat nach Anspruch 1, dadurch gekennzeichnet, daß als Komponente B Ketanserin eingesetzt wird.

6. Kombinationspräparat nach Ansprüchen 1 bis 6, dadurch gekennzeichnet, daß sie auf 1 Gewichtsteil der Komponente A 0,01 bis 100 Gewichtsteile der Komponente B enthalten.

7. Verfahren zur Herstellung eines Kombinationspräparates nach Anspruch 1 dadurch gekennzeichnet, daß man die Einzelkomponenten in diese aufhörende inerte Lösemittel auflöst und gegebenenfalls nach Abdampfen des Lösemittels die Kombinationen in üblicher Weise mit Hilfsmitteln vermischt.

8. Verwendung der Kombination nach Anspruch 1 zur Behandlung von cerebralen Ischämien.
